# EUROPÄISCHE PATENTSCHRIFT

(11) **EP 2 039 439 B1**
(45) Veröffentlichungstag und Bekanntmachung des Hinweises auf die Patenterteilung: **19.05.2010**
(21) Anmeldenummer: 08164555.8
(22) Anmeldetag: 18.09.2008
(51) Int. Cl.: B08B 7/00

(54) **Vorrichtung zum Desinfizieren von Behältnisverschlüssen**
Device for disinfecting container fasteners
Dispositif et désinfection de fermetures de récipients

(30) Priorität: 20.09.2007 DE 102007045142
(43) Veröffentlichungstag der Anmeldung: 25.03.2009
(73) Patentinhaber: Krones AG, 93073 Neutraubling (DE)
(72) Erfinder: Buchhauser, Klaus, 93180 Deuerling (DE); Frankenberger, Günter, 93096 Köfering (DE); Klepatz, Sebastian, 93057 Regensburg (DE)
(74) Vertreter: Bittner, Bernhard

(56) Entgegenhaltungen:
- WO-A-2007/147535
- DE-A1- 10 145 102
- DE-U1- 29 708 145

## Beschreibung

Die vorliegende Erfindung bezieht sich auf eine Vorrichtung zum Desinfizieren von Verschlüssen und insbesondere von Behältnisverschlüssen. Bei der Getränkeherstellung ist es üblich, nicht nur die Behältnisse selbst vor deren Abfüllung zu sterilisieren und zu reinigen, sondern auch die Verschlüsse für die Behältnisse. Eine derartige Reinigung kann beispielsweise über eine Reinigungsflüssigkeit erfolgen, wobei die Verschlüsse in einem entsprechenden Flüssigkeitsbad geführt werden.

Aus der DE 297 08 145 U1 ist eine Vorrichtung zum Reinigen und/oder Sterilisieren von Verschlusskappen bekannt. Dabei werden die Verschlusskappen durch einen Kanal transportiert und dabei mit Flüssigkeit beaufschlagt. Dieser Kanal ist dabei in Form einer im Wesentlichen horizontalen Spirale ausgeführt, die sich durch ein Tauchbad erstreckt.

Die DE 101 45 102 A1 beschreibt ein Verfahren und eine Vorrichtung zum Sterilisieren von Verschlusskappen für Behälter.

Die WO 2007/147535 A beschreibt eine Vorrichtung gemäß dem Oberbegriff von Anspruch 1.

In vielen Bereichen ist es jedoch wünschenswert, die Reinigung dieser Verschlüsse trocken durchzuführen, d. h. auf ein entsprechendes Tauchbad sollte verzichtet werden. Der vorliegenden Erfindung liegt daher die Aufgabe zugrunde, eine Vorrichtung zum Reinigen von Behältnisverschlüssen zur Verfügung zu stellen, welche eine trockene Reinigung bzw. eine Reinigung ohne ein flüssiges Reinigungsmittel ermöglicht. Dies wird erfindungsgemäß durch die Gegenstände der unabhängigen Ansprüche 1, 12 und 15 erreicht. Vorteilhafte Ausführungsformen und Weiterbildungen sind Gegenstand der Unteransprüche.

Eine erfindungsgemäße Vorrichtung zum Desinfizieren von Behältnisverschlüssen weist ein Gehäuse auf, sowie einen innerhalb dieses Gehäuses angeordneten Transportpfad bzw. Transportkanal, entlang dessen die Verschlüsse transportiert werden. Dabei weist dieser Transportpfad wenigstens abschnittsweise eine spiralförmige Gestalt auf. Erfindungsgemäß weist die Vorrichtung eine Zuführeinrichtung auf, um dem Gehäuse ein gasförmiges Medium zur Desinfektion der Verschlüsse zuzuführen und es ist eine gegenüber diesem Gehäuse bewegbare Verschiebeeinrichtung vorgesehen, welche die Verschlüsse entlang des spiralförmigen Transportpfads transportiert. Vorzugsweise ist das Gehäuse gasdicht ausgeführt, zumindest in demjenigen Bereich, in dem der Transportpfad verläuft.

Bei einer weiteren vorteilhaften Ausführungsform weist das Gehäuse eine den Transportpfad umgebende Gehäusewandung auf, in der eine Vielzahl von Zuführeinrichtungen in Form von Düsen angeordnet sind.

Bei einer weiteren vorteilhaften Ausführungsform weist die Verschiebeeinrichtung eine Vielzahl von Verschiebekörpern auf, die drehbar um eine gemeinsame Achse angeordnet sind. Durch diese Verschiebekörper werden durch die einzelnen Behältnisverschlüsse entlang des oben erwähnten Transportpfads geführt.

Bei einer weiteren vorteilhaften Ausführungsform ist der Transportpfad derart ausgeführt, dass die Verschlüsse entlang der Spirale von außen nach innen transportiert werden. In einer derartigen Ausführungsform kann besonders vorteilhaft ein Einlaufstern an dem Außenumfang der Spirale angeordnet sein, der die einzelnen Verschlüsse dem spiralförmigen Pfad zuführt.

Bei einer weiteren vorteilhaften Ausführungsform sind die Verschiebekörper an einem gemeinsamen Träger angeordnet. Vorteilhaft weisen die Schiebekörper einen in der Bewegungsrichtung gekrümmten äußeren Abschnitt und besonders bevorzugt auch einen gegenüber der radialen Richtung der Verschiebekörper geneigten Bereich auf. Auf diese Weise werden die einzelnen Behältnisverschlüsse während der Verschiebebewegung geringfügig radial nach innen gedrängt, sodass Verkantungen der Behältnisverschlüsse innerhalb des Transportpfads verhindert werden können.

Vorzugsweise sind die Verschiebekörper an einem gemeinsamen Träger angeordnet. Dieser gemeinsame Träger ist wiederum drehbar bezüglich einer Drehachse, die besonders bevorzugt im Wesentlichen im Zentrum des spiralförmigen Transportpfads verläuft, angeordnet.

Dies bedeutet, dass sich alle Verschiebekörper mit der gleichen Drehgeschwindigkeit gegenüber dem Gehäuse bewegen. Dabei ist es möglich, dass der Transportpfad durch die Verschiebekörper so begrenzt wird, dass zwischen zwei Verschiebkörpern jeweils ein Behältnisverschluss geführt wird. Es wäre jedoch auch möglich, dass von einem Verschiebekörper mehrere Behältnisverschlüsse geführt werden.

Bei einer weiteren vorteilhaften Ausführungsform weist die Vorrichtung eine Trägereinrichtung auf, entlang derer die Verschlüsse geschoben werden und der Transportpfad wird durch diese Trägereinrichtung und eine spiralförmig gekrümmte Führungseinrichtung gebildet. Vorzugsweise handelt es sich dabei bei der Trägereinrichtung um einen Boden, auf dem die Verschlüsse aufliegen und entlang dessen sie bewegt werden. Die spiralförmig gekrümmte Führungseinrichtung ist dabei vorzugsweise eine sich senkrecht bezüglich dieses Bodens erstreckende Wand, die spiralförmig verläuft, um die Behältnissverschlüsse entlang dieser Wand führen zu können. Vorzugsweise ist die Führungseinrichtung gegenüber der Trägereinrichtung in einer senkrecht zu der Trägereinrichtung stehenden Richtung beabstandet, und besonders bevorzugt liegt der Abstand in einem Bereich von 4 - 6 mm.

Vorzugsweise weist das Gehäuse auch einen Einlaufbereich auf, über welchen die Behältnisverschlüsse zugeführt werden sowie einen Auslaufbereich, über welchen die Behältnisverschlüsse abgeführt werden. Vorzugsweise ist wenigstens in dem Auslaufbereich oder in dem Einlaufbereich eine Absaugeinrichtung für das gasförmige Medium vorgesehen. Besonders bevorzugt ist sowohl in dem Auslaufbereich als auch in dem Einlaufbereich eine Absaugeinrichtung für das gasförmige Medium angeordnet.

Bei einer weiteren vorteilhaften Ausführungsform sind die Verschiebekörper derartig angeordnet, dass sie die Verschlüsse an einem Grundkörper derselben kontaktieren. Umgekehrt werden damit die Verschlusskörper nicht an deren Spitzen kontaktiert. Auf diese Weise ist die Vorrichtung auch für Behältnisverschlüsse mit unterschiedlichen Höhen geeignet.

Bei einer weiteren vorteilhaften Ausführungsform schließt sich an dem Transportpfad eine Transportrinne mit einem vorzugsweise S-förmigen Profil an. Über diese Transportrinne werden die Behältnisverschlüsse aus der Vorrichtung abgeführt.

Bei einer weiteren vorteilhaften Ausführungsform ist an dem Gehäuse und oberhalb des Gehäuses ein schwenkbarer Deckel angeordnet. Dieser Deckel kann geöffnet werden, um dem Benutzer Zugang zu den einzelnen Behältnisverschlüssen zu verschaffen.

Bei einer weiteren vorteilhaften Ausführungsform ist in dem Gehäuse eine Vielzahl von Reinigungsdüsen zur Reinigung des Gehäuses angeordnet. Dabei kann es sich um Reinigungsdüsen handeln, die außerhalb des normalen Betriebs eine Innenreinigung des Gehäuses, beispielsweise auch mit Flüssigkeiten, erlauben. Vorzugsweise ist an dem Gehäuse eine Heizeinrichtung für das gasförmige Medium vorgesehen. Vorteilhaft werden dabei die Behältnisverschlüsse durch das Gehäuse transportiert, in welchem gasförmiges Wasserstoffperoxid bei Temperaturen von zwischen 40° und 50° C ca. 20 - 30 Sek. auf die Behältnisverschlüsse einwirkt. Der Auslauf dieses Gehäuses befindet sich, wie oben erwähnt, bereits in einem Reinraum. Die Heizeinrichtung bewirkt, dass das gasförmige Medium, d. h. insbesondere das Wasserstoffperoxid, vor dem Eintritt in das Gehäuse erwärmt wird.

Die vorliegende Erfindung bezieht sich weiterhin auf eine Anordnung zum Desinfizieren von Behältnisverschlüssen mit einer Vorrichtung der oben beschriebenen Art sowie einer Zuführeinrichtung, welche die Verschlüsse dem Transportpfad zuführt. Erfindungsgemäß weist die Zuführeinrichtung ein Transportrad auf, welches die Verschlüsse dem Transportpfad einzeln zuführt. Damit wird erreicht, dass die Verschlüsse in einer genau definierten Weise an die Verschiebeeinrichtung bzw. die einzelnen Verschiebekörper übergeben werden.

Vorzugsweise ist das Transportrad in dem Gehäuse der Vorrichtung untergebracht.

Weiteren vorteilhaften Ausführungsformen ist in denjenigen Bereich des Gehäuses, in dem der Transportpfad vorgesehen ist, das gasförmige Medium wenigstens zeitweise unter einem höheren Druck als an in denjenigen Bereich des Gehäuses, in dem das Transportrad angeordnet ist. Damit findet ein Druckgefälle von dem Bereich des Transportpfades hin zu dem Bereich des Transportrades statt, wodurch das gasförmige Medium in definierter Weise aus dem Gehäuse ausgeführt werden kann.

Die vorliegende Erfindung ist weiterhin auf ein Verfahren zum Reinigen von Behältnisverschlüssen gerichtet, wobei die Behältnisse während des Reinigungsvorgangs entlang eines innerhalb eines Gehäuses angeordneten Transportpfads geführt werden, und wobei der Transportpfad wenigstens abschnittsweise eine spiralförmige Gestalt aufweist. Erfindungsgemäß wird den Behältnisverschlüssen während des Transports auf dem Transportpfad innerhalb des Gehäuses ein gasförmiges Medium zur deren Sterilisation zugeführt. Hierbei werden die Behältnisverschlüsse entlang des spiralförmigen Transportpfads durch eine gegenüber dem Gehäuse bewegbare Verschiebeeinrichtung transportiert.

Weitere Vorteile und Ausführungsformen ergeben sich aus den beigefügten Zeichnungen:

Darin zeigen:
- Fig. 1: eine Gesamtdarstellung einer erfindungsgemäßen Anordnung zum Desinfizierten von Behältnisverschlüssen;
- Fig. 2: eine teilgeschnittene Ansicht der Anordnung aus Fig. 1;
- Fig. 3: eine Draufsicht auf eine erfindungsgemäße Vorrichtung zum Desinfizieren von Behältnisverschlüssen und
- Fig. 4: eine vergrößerte Ansicht eines Teilbereichs der Ansicht aus Fig. 3.

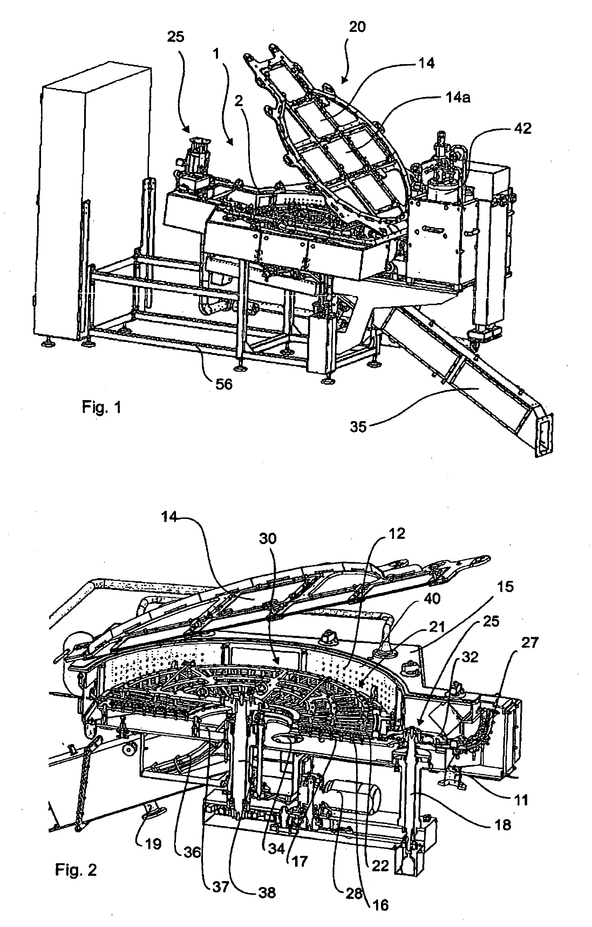

Fig. 1 zeigt eine erfindungsgemäße Anordnung 20 zum Desinfizieren von Behältnisverschlüssen. Diese Anordnung 20 weist eine Gestell 56 auf, auf dem ein Gehäuse 2 für die Vorrichtung 1 zum Desinfizieren von Behältnisverschlüssen angeordnet ist. Das Bezugszeichen 14 bezieht sich auf einen Deckel, mit dem das Gehäuse 2 gasdicht abgedeckt bzw. verschlossen werden kann. Dieser Deckel 14 ist dabei schwenkbar an dem Gehäuse 2 angeordnet und weist Sichtfenster 14a auf. Dabei ist es bevorzugt auch möglich, dass einzelne dieser Sichtfenster 14a separat geöffnet werden können. Das Öffnen und Schließen des Deckels 14 erfolgt pneumatisch. Eine Öffnung mittels Seilwinde ist auch vorstellbar.

Ausgehend von einem Reservoir 42 wird dem Gehäuse 2 ein gasförmiges Medium, wie beispielsweise Wasserstoffperoxid, zugeführt. Über eine Zuführeinrichtung 25 können dem Gehäuse 2 die Behältnisverschlüsse zugeführt werden. Innerhalb des Gehäuses 2 werden die Behältnisverschlüsse entlang eines unten genauer dargestellten Transportpfads transportiert und zwar bei der in den Figuren gezeigten Ausführungsform von außen nach innen und gelangen schließlich über eilen Abführkanal 35 aus der Anordnung hinaus.

Fig. 2 zeigt eine teilgeschnittene Ansicht der Anordnung 20 aus Figur 1. Man erkennt, dass die Zuführeinrichtung 25 eine Verschlusszuführungsrinne 27 aufweist, welche die Behältnisverschlüsse an einen Eintaktstern 32 übergibt. Dabei kann diese Zuführung der Verschlüsse luftunterstützt erfolgen. Dieser Eintaktstern 32 transportiert die Verschlüsse horizontal weiter an eine in ihrer Gesamtheit mit 30 bezeichnete Spiralbahn, welche den Transportpfad 4 bildet. In dem Bereich dieser Spiralbahn 30 übernimmt eine Verschiebeeinrichtung 21 in Form eines Rotors die Bewegung bzw. Führung der Verschlüsse. Dabei ist eine angetriebene Welle vorgesehen, welche die in ihrer Gesamtheit mit 21 gekennzeichnete Verschiebeeinrichtung dreht. An dieser Verschiebeeinrichtung sind unten im Detail erläuterte Verschiebskörper vorgesehen, welche die Behältnisverschlüsse schieben. Das Gehäuse 2 weist eine Umfangswandung 40 auf, in der eine Vielzahl von Zuführöffnungen 12 angeordnet sind, über welche das Gas in das Gehäuse 2 geführt werden kann.

Der Eintaktstern 32 weist ebenfalls eine Antriebswelle 18 auf, welche diesen Eintaktstern 32 rotatorisch antreibt. Eine Antriebseinrichtung, wie ein Motor 28, treibt bei dieser Ausführungsform sowohl die Welle 38 als auch die Welle 18 synchron an. In dem Eintaktbereich zwischen dem Eintaktstern 32 und dem Transportpfad 4 werden die Behältnisverschlüsse allseitig geführt.

Das Bezugszeichen 17 bezieht sich auf eine Trägereinrichtung in Form eines Bodenblechs. Auf der Spiralbahn 30 stehen die Behältnisverschlüsse auf diesem vorzugsweisen geschlitzten Bodenblech 17 und das Bezugszeichen 16 bezieht sich auf eine Führungseinrichtung, die hier als spiralförmiges Blech ausgeführt ist, welches in einem vorgegebenen Abstand, beispielsweise 5 mm, über der Trägereinrichtung 17 angeordnet ist. Das Bezugszeichen 22 bezieht sich auf einen einzelnen Verschiebekörper, wobei eine Vielzahl derartiger Verschiebekörper 22 vorgesehen ist, die sich, wie oben gesagt, jeweils in radialer Richtung erstrecken. Die Führungseinrichtung 16 gibt damit die Bewegungsrichtung vor. Der oberhalb der Führungseinrichtung angeordnete Träger bzw. Rotor 24 mit den Verschiebekörpern 22 übernimmt das eigentliche Verschieben der Behältnisverschlüsse. Dabei werden die Behältnisverschlüsse jeweils an ihrem Grundkörper für die Verschiebung berührt. Diese Gestaltung weist den Vorteil auf, dass der Verschluß horizontal auf einer Spiralbahn hier zur Maschinenmitte wandert, nach oben ist der Verschluß frei.

Auf diese Weise können durch die Führungseinrichtung 16 Verschlüsse mit verschieden gestalteten Staubkappen oder auch ohne Staubkappen transportiert werden, ohne dass hierzu die Einheiten verändert werden müssen. Vorausgesetzt werden jeweils nur ein identischer oder zumindest ähnlich gestalteter Grundkörper der Behältnisverschlüsse.

Diese erwähnten Transporteinheiten, das heißt sowohl der Eintaktstern 32 als auch die Verschiebeeinrichtung 21 sind in dem gasdichten Gehäuse 2 untergebracht. Die Wellen 18 und 38 sind bevorzugt jeweils mit Überlastkupplungen ausgestattet. Bei dem Motor 28 handelt es sich vorzugsweise um einen zentral angeordneten Getriebemotor.

Durch die gleichmäßige Anordnung der Zuführöffnungen 12 ist es möglich, das gasförmige Medium gleichmäßige über das gesamte Gehäuse 2 zu verteilen. Weiterhin sind in dem Gehäuse eine Vielzahl von (nicht im Detail gezeigten) Reinigungsdüsen vorgesehen, welche zur Innenreinigung des Gehäuses dienen. Der Abfluss für das Reinigungsmittel erfolgt über eine Öffnung 34 bzw. eine Drainageverrohrung, welche mit Klappenventilen geschlossen werden kann. Insgesamt weist das Gehäuse 2 drei derartige Öffnungen 34 sowie eine (vorzugsweise rechteckige) Abführöffnung 37 für die Behältnisverschlüsse auf.

An dem Außenumfang des Gehäuses sind Gaseintrittskammern 15 vorgesehen, über die das H₂O₂-Luftgemisch in den Behandlungsraum, das heißt das Gehäuse 2, eingegast wird. Die Gasgeschwindigkeit selbst sowie auch Verwirbelungen, die durch die Drehung der Verschiebeeinrichtung 21 erzeugt werden, bewirken eine gleichmäßige Verteilung des Gases in der Kammer und auch in dem Inneren der einzelnen Verschlüsse. Dies ist für eine Entkeimung vorteilhaft. Das einströmende Gas kann über ein Absaugrohr 11 in dem Einlaufbereich bzw. der Zuführeinrichtung 25 sowie über ein Rohr 19 in der Abführeinrichtung 35 abgesaugt werden.

Während des Betriebs herrscht in der Behandlungskammer, das heißt demjenigen Bereich des Gehäuses 2, in dem der Transportpfad 4 als spiralförmige Bahn angeordnet ist, ein leichter Überdruck und in dem Einlaufbereich, das heißt demjenigen Bereich des Gehäuses, in dem der Eintaktstern 32 vorgesehen ist, wird mittels Unterdruck ein Austreten von H₂O₂ in die Umgebung verhindert. Ein Gegendruck in dem Bereich des stromabwärts angeordneten (nicht gezeigten) Füllers verhindert das Eindringen von H₂O₂ in den Füllerbereich. Die erforderliche Behandlungstemperatur des H₂O₂-Luftgemisches wird durch die Temperatur des einströmenden H₂O₂-Luftgemisches sowie mittels an dem Gehäuse 2 angeordneter Heizdecken (nicht gezeigt) gehalten. Weiterhin sind vorzugsweise Druck- und Temperatursensoren vorgesehen, welche die Betriebszustände laufend überwachen.

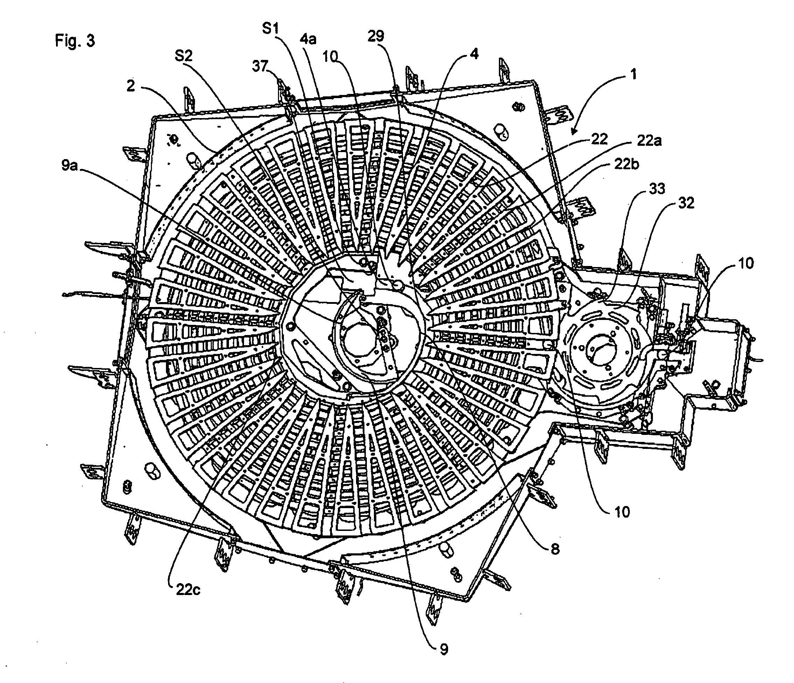

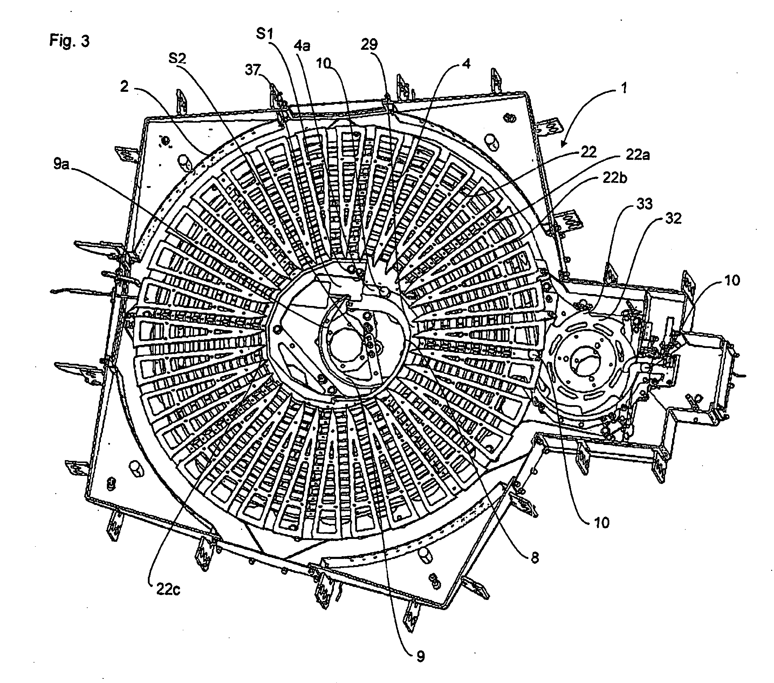

Fig. 3 zeigt eine Draufsicht auf eine erfindungsgemäße Vorrichtung 1. Dabei sind die jeweiligen Antriebswellen für den Eintaktstern 32 und der Verschiebeeinrichtung 21 nicht dargestellt. Dem Eintaktstern 32 werden die Behältnisverschlüsse 10 über die oben erwähnte Rinne 27 zugeführt. Der Eintaktstern 32 weist eine Vielzahl von sägezahnförmigen Ausnehmungen 33 auf, die zur Führung der Behältnisverschlüsse 10 dienen. Der Eintaktstern 32 übergibt die Behältnisverschlüsse 10 an die Verschiebeeinrichtung 21, was ebenfalls in Fig. 3 dargestellt ist.

Die Verschiebeinrichtung 21 weist, wie oben erwähnt, eine Vielzahl von Verschiebekörpern 22 auf, die sich jeweils radial nach außen erstrecken. Diese Verschiebekörper 22 weisen die Gestalt eines sich radial nach innen verjüngenden Dreiecks auf. Auf diese Weise wird erreicht, dass die Abstände zwischen einzelnen Führungskanten 22a und 22b von außen nach innen jeweils im Wesentlichen konstant bleiben und damit jeweils einen Behältnisverschluß 10 zwischen den einzelnen Verschiebekörpern 22 mit definiertem Spiel aufgenommen werden kann. Weiterhin weisen die Verschiebekörper radial innen liegende Verjüngungen auf bzw. Schrägflächen 22c auf, die derart geneigt sind, dass im Inneren des Transportpfades 4 die Behältnisverschlüsse auch nach innen gedrängt werden.

Das Bezugszeichen 4 bezieht sich damit in seiner Gesamtheit auf den spiralförmig von außen nach innen verlaufenden Transportpfad entlang dessen die Behältnisverschlüsse 10 geführt werden. Am Ende dieses Transportpfades 4 ist ein Bereich 4a vorgesehen, entlang dessen die Behältnisverschlüsse 10 bis zu der rechtecksförmigen Abführöffnung 37 geführt werden, über die sie aus dem Gehäuse 2 abgeführt werden. An dem Ende des Transportpfades 4 werden die Behältnisse 10 über eine s-förmige Transferrinne 36 (vgl. Figur 2) übergeben. Über diese Rinne gleiten die Behältnisverschlüsse evtl. luftunterstützt zu dem (nicht gezeigten) Verschließer.

Das Bezugszeichen 8 bezieht sich auf einen ersten Schwenkhebel, der gegenüber einem Schwenkpunkt S1 schwenkbar angeordnet ist und das Bezugszeichen 9 auf einen zweiten Schwenkhebel, der gegenüber einem Schwenkpunkt S2 schwenkbar angeordnet ist. Diese beiden Schwenkhebel 8 und 9 dienen zur Störungsbehebung und sind von außen manuell zu bedienen. Der Außenumfang des ersten Schwenkhebels 8 dient als Führung für die Behältnisverschlüsse 10. Falls es zu einem Stau der Behältnissverschlüsse kommt, kann dieser erste Schwenkhebel 8 im Urzeigersinn um die Schwenkachse S1 geklappt werden und gibt damit den Weg nach radial innen für die Behältnisverschlüsse frei.

Falls sich auf diese Weise noch nicht alle Behältnisverschlüsse 10 im Zentralbereich des Gehäuses 2 sammeln, kann noch ein zweiter Schwenkhebel 9 hier um den Schwenkpunkt S2 ebenfalls im Urzeigersinn geschwenkt werden, damit dessen Spitze in die Transportbahn 4 eingreift. Die Innenwandung 9a dieses zweiten Schwenkhebels 9 dient dann als Führung für die Behältnisverschlüsse 10, so dass die Behältnisverschlüsse 10 auch auf diese Weise in das Zentrum des Gehäuses 2 gedrängt werden.

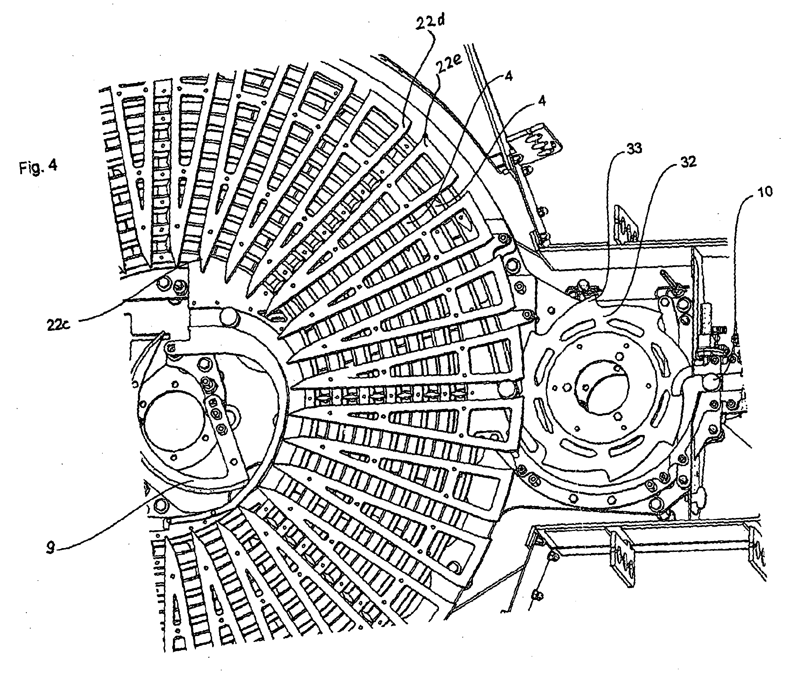

Fig. 4 zeigt eine detailliertere Ansicht der Darstellung aus Fig. 3. Man erkennt auch hier den Transportpfad 4, der sich spiralförmig von außen nach innen erstreckt. Auch erkennt man an der Trägereinrichtung 17 eine Führungsrinne 29 für die Behältnisverschlüsse 10. Auch erkennt man wiederum die Spitzen 22c der Verschiebekörper 22, die in dem innenliegenden Bereich die Behältnisverschlüsse ebenfalls nach radial innen, das heißt in Richtung des ersten Schwenkhebels 8 drängen. An ihrem radial außen begrenzenden Bereich weisen die Verschiebekörper 22 einen in der Drehrichtung der Verschiebekörper 22 gekrümmten Abschnitt 22d auf sowie einen entsprechend gekrümmten Bereich 22e an dem jeweils nachfolgenden Verschiebekörper.

## Patentansprüche

1. Vorrichtung (1) zum Desinfizieren von Behältnisverschlüssen (10) mit einem Gehäuse (2), mit einem innerhalb des Gehäuses (2) angeordneten Transportpfad (4), entlang dessen die Verschlüsse (10) transportiert werden, wobei der Transportpfad (4) wenigstens abschnittsförmig eine spiralförmige Gestalt aufweist,
wobei die Vorrichtung (1) eine Zuführeinrichtung (12) aufweist, um dem Gehäuse (2) ein gasförmiges Medium zur Desinfektion der Behältnisverschlüsse (10) zuzuführen, **dadurch gekennzeichnet, dass**
eine gegenüber dem Gehäuse bewegbare Verschiebeeinrichtung (21) vorgesehen ist, welche die Behältnisverschlüsse (10) entlang des spiralförmigen Transportpfads (4) transportiert.

2. Vorrichtung (1) nach Anspruch 1,
**dadurch gekennzeichnet, dass**
die Verschiebeeinrichtung (21) eine Vielzahl von Verschiebekörpern (22) aufweist, die drehbar um eine gemeinsame Achse angeordnet sind.

3. Vorrichtung (1) nach wenigstens einem der vorangegangenen Ansprüche,
**dadurch gekennzeichnet, dass**
der Transportpfad (4) derart ausgeführt ist, dass die Behältnisverschlüsse (10) von außen nach innen transportiert werden.

4. Vorrichtung (1) nach wenigstens einem der vorangegangenen Ansprüche,
**dadurch gekennzeichnet, dass**
die Verschiebekörper (22) an einem gemeinsamen Träger (24) angeordnet sind.

5. Vorrichtung nach wenigstens einem der vorangegangenen Ansprüche,
**dadurch gekennzeichnet, dass**
die Vorrichtung eine Trägereinrichtung (17) aufweist, entlang derer die Verschlüsse (10) verschoben werden und der Transportpfad (4) durch diese Trägereinrichtung (17) und eine spiralförmig gekrümmte Führungseinrichtung (16) gebildet wird.

6. Vorrichtung (1) nach Anspruch 5,
**dadurch gekennzeichnet, dass**
die Führungseinrichtung (16) gegenüber der Trägereinrichtung (17) beabstandet ist.

7. Vorrichtung (1) nach wenigstens einem der vorangegangenen Ansprüche,
**dadurch gekennzeichnet, dass**
die Verschiebekörper (22) derartig angeordnet sind, dass sie die Behältnisverschlüsse (10) an einem Grundkörper derselben kontaktieren.

8. Vorrichtung (1) nach wenigstens einem der vorangegangenen Ansprüche,
**dadurch gekennzeichnet, dass**
sich an den Transportpfad (4) eine Transportrinne (36) mit einem S - förmigen Profil anschließt.

9. Vorrichtung nach wenigstens einem der vorangegangenen Ansprüche,
**dadurch gekennzeichnet, dass**
an dem Gehäuse (2) und oberhalb des Gehäuses ein schwenkbarer Deckel (14) angeordnet ist.

10. Vorrichtung (1) nach wenigstens einem der vorangegangenen Ansprüche,
**dadurch gekennzeichnet, dass**
in dem Gehäuse (2) eine Vielzahl von Reinigungsdüsen zur Reinigung des Gehäuses (2) angeordnet ist.

11. Vorrichtung (1) nach wenigstens einem der vorangegangenen Ansprüche,
**dadurch gekennzeichnet, dass**
an dem Gehäuse (2) eine Heizeinrichtung für das gasförmige Medium vorgesehen ist.

12. Anordnung (20) zum Desinfizieren von Behältnisverschlüssen (10) mit einer Vorrichtung (1) nach wenigstens einem der vorangegangenen Ansprüche und einer Zuführeinrichtung (25), welche die Behältnisverschlüsse (10) dem Transportpfad (4) zuführt,
**dadurch gekennzeichnet, dass**
die Zuführeinrichtung (25) ein Transportrad (32) aufweist, welches die Behältnisverschlüsse (10) dem Transportpfad (4) vereinzelt zuführt.

13. Anordnung (20) nach Anspruch 12,
**dadurch gekennzeichnet, dass**
das Transportrad (32) in dem Gehäuse (2) der Vorrichtung (1) untergebracht ist.

14. Anordnung (20) nach Anspruch 13,
**dadurch gekennzeichnet, dass**
in demjenigen Bereich des Gehäuses (2), in dem der Transportpfad (4) vorgesehen ist, das gasförmige Medium wenigstens zeitweise unter einem höheren Druck steht als in dem demjenigen Bereich des Gehäuses (2), in dem das Transportrad (32) angeordnet ist.

15. Verfahren zum Reinigen von Behältnisverschlüssen (10), wobei die Behältnisse (10) während des Reinigungsvorgangs entlang eines innerhalb eines Gehäuses (2) angeordneten Transportpfads (4) geführt werden, wobei der Transportpfad (4) wenigstens abschnittsweise eine spiralförmige Gestalt aufweist,
wobei den Behältnisverschlüssen (10) während des Transports auf dem Transportpfad (4) innerhalb des Gehäuses (2) ein gasförmiges Medium zu deren Sterilisation zugeführt wird,
**dadurch gekennzeichnet, dass**
die Behältnisverschlüsse (10) entlang des spiralförmigen Transportpfads (4) durch eine gegenüber dem Gehäuse (2) bewegbare Verschiebeeinrichtung (21) transportiert werden.

## Claims

1. Apparatus (1) for disinfecting container closures (10), comprising a housing (2) and comprising a transport path (4) which is arranged inside the housing (2) and along which the closures (10) are transported, wherein the transport path (4) has at least partially a spiral configuration, wherein the apparatus (1) comprises a supply device (12) for supplying to the housing (2) a gaseous medium for disinfecting the container closures (10), **chracterised in that** a displacement device (21) is provided which can move with respect to the housing and which transports the container closures (10) along the spiral transport path (4).

2. Apparatus (1) according to claim 1, **characterised in that** the displacement device (21) comprises a large number of displacement bodies (22) which are arranged such that they can rotate around a common axis.

3. Apparatus (1) according to at least one of the preceding claims, **characterised in that** the transport path (4) is designed in such a way that the container closures (10) are transported from the outside towards the inside.

4. Apparatus (1) according to at least one of the preceding claims, **characterised in that** the displacement bodies (22) are arranged on a common support (24).

5. Apparatus according to at least one of the preceding claims, **characterised in that** the apparatus comprises a support device (17) along which the closures (10) are displaced, and the transport path (4) is formed by this support device (17) and a spirally curved guide device (16).

6. Apparatus (1) according to claim 5, **characterised in that** the guide device (16) is arranged at a distance from the support device (17).

7. Apparatus (1) according to at least one of the preceding claims, **characterised in that** the displacement bodies (22) are arranged in such a way that they contact the container closures (10) on a base body thereof.

8. Apparatus (1) according to at least one of the preceding claims, **characterised in that** the transport path (4) is adjoined by a transport chute (36) with an S-shaped profile.

9. Apparatus according to at least one of the preceding claims, **characterised in that** a pivotable cover (14) is arranged on the housing (2) and above the housing.

10. Apparatus (1) according to at least one of the preceding claims, **characterised in that** a large number of cleaning nozzles for cleaning the housing (2) are arranged inside the housing (2).

11. Apparatus (1) according to at least one of the preceding claims, **characterised in that** a heating device for the gaseous medium is provided on the housing (2).

12. Arrangement (20) for disinfecting container closures (10), comprising an apparatus (1) according to at least one of the preceding claims and a feed device (25) which feeds the container closures (10) to the transport path (4), **characterised in that** the feed device (25) comprises a transport wheel (32) which feeds the container closures (10) individually to the transport path (4).

13. Arrangement (20) according to claim 12, **characterised in that** the transport wheel (32) is accommodated in the housing (2) of the apparatus (1).

14. Arrangement (20) according to claim 13, **characterised in that** the gaseous medium is at least temporarily at a higher pressure in the region of the housing (2) in which the transport path (4) is provided than in the region of the housing (2) in which the transport wheel (32) is arranged.

15. Method for cleaning container closures (10), wherein the containers (10) during the cleaning operation are guided along a transport path (4) arranged inside a housing (2), wherein the transport path (4) has at least partially a spiral configuration, **characterised in that** a gaseous medium is supplied to the container closures (10) for sterilisation purposes during transport on the transport path (4) inside the housing (2).

## Revendications

1. Dispositif (1) de désinfection de fermetures (10) de récipients, avec un carter (2), avec un chemin (4) de transport disposé à l'intérieur du carter (2), sur lequel sont transportées les fermetures (10), ledit chemin (4) de transport présentant au moins partiellement une forme en spirale,
le dispositif (1) comportant un système (12) d'alimentation destiné à amener au carter (2) un milieu gazeux pour la désinfection des fermetures (10) de récipients,
**caractérisé en ce**
**qu'**il est prévu un mécanisme (21) de déplacement mobile par rapport au carter, lequel transporte les fermetures (10) de récipients le long du chemin (4) de transport en forme de spirale.

2. Dispositif (1) selon la revendication 1,
**caractérisé en ce que**
le mécanisme (21) de déplacement comporte une pluralité de corps (22) de déplacement rotatifs autour d'un axe commun.

3. Dispositif (1) selon au moins une des revendications précédentes,
**caractérisé en ce que**
le chemin (4) de transport est réalisé de telle manière à transporter les fermetures (10) de récipients de l'extérieur vers l'intérieur.

4. Dispositif (1) selon au moins une des revendications précédentes,
**caractérisé en ce que**
les corps (22) de déplacement sont disposés contre un support (24) commun.

5. Dispositif (1) selon au moins une des revendications précédentes,
**caractérisé en ce que**
le dispositif comporte un dispositif (17) de support le long duquel les fermetures (10) sont déplacées et **en ce que** le chemin (4) de transport est formé par ledit dispositif (17) de support et un dispositif (16) de guidage incurvé en forme de spirale.

6. Dispositif (1) selon la revendication 5,
**caractérisé en ce que**
le dispositif (16) de guidage est espacé du dispositif (17) de support.

7. Dispositif (1) selon au moins une des revendications précédentes,
**caractérisé en ce que**
les corps (22) de déplacement sont disposés de telle manière à être en contact avec les fermetures (10) de récipients sur un corps de base de ceux-ci.

8. Dispositif (1) selon au moins une des revendications précédentes,
**caractérisé en ce**
**qu'**une goulotte (36) de transport à profil en S succède au chemin (4) de transport.

9. Dispositif selon au moins une des revendications précédentes, **caractérisé en ce**
**qu'**un couvercle (14) pivotant est disposé contre le carter (2) et au-dessus du carter.

10. Dispositif (1) selon au moins une des revendications précédentes,
**caractérisé en ce**
**qu'**une pluralité de buses de nettoyage, destinées au nettoyage du carter (2), sont disposées dans le carter (2).

11. Dispositif (1) selon au moins une des revendications précédentes,
**caractérisé en ce**
**qu'**un dispositif de chauffage pour le milieu gazeux est prévu sur le carter (2).

12. Système (20) pour la désinfection de fermetures (10) de récipients, avec un dispositif (1) selon au moins une des revendications précédentes et un dispositif (25) d'amenée amenant les fermetures (10) de récipients vers le chemin (4) de transport,
**caractérisé en ce que**
le dispositif (25) d'amenée comporte une roue (32) de transport amenant individuellement chaque fermeture (10) de récipient vers le chemin (4) de transport.

13. Système (20) selon la revendication 12,
**caractérisé en ce que**
la roue (32) de transport est mise en place dans le carter (2) du dispositif (1).

14. Système (20) selon la revendication 13,
**caractérisé en ce que,**
dans la partie du carter (2) où est prévu le chemin (4) de transport, le milieu gazeux est soumis au moins temporairement à une pression supérieure à celle régnant dans la partie du carter (2) où est disposée la roue (32) de transport.

15. Procédé de nettoyage de fermetures (10) de récipients, où, pendant le processus de nettoyage, les fermetures (10) de récipients sont amenées le long d'un chemin de transport (4) disposé à l'intérieur d'un carter (2), ledit chemin (4) de transport présentant au moins partiellement une forme en spirale,
où un milieu gazeux pour la désinfection des fermetures (10) de récipients est amené vers celles-ci pendant leur transport sur le chemin (4) de transport à l'intérieur du carter (2),
**caractérisé en ce que**
les fermetures (10) de récipients sont transportées le long du chemin (4) de transport en forme de spirale par un mécanisme (21) de déplacement mobile par rapport au carter (2).
